# EUROPEAN PATENT SPECIFICATION

(11) **EP 4 275 494 B1**
(45) Date of publication and mention of the grant of the patent: **04.12.2024**
(21) Application number: 23172941.9
(22) Date of filing: 11.05.2023
(51) Int. Cl.: A01N 63/28, C12N 1/20

(54) **STREPTOMYCES STRAIN, COMPOSITION COMPRISING SAID STRAIN AND METHOD OF CULTIVATION OF PLANTS USING THE COMPOSITION**
STREPTOMYCES-STAMM, ZUSAMMENSETZUNG, DIE DEN STAMM ENTHÄLT, UND VERFAHREN ZUR PFLANZENZUCHT UNTER VERWENDUNG DER ZUSAMMENSETZUNG
SOUCHE DE STREPTOMYCES, COMPOSITION CONTENANT LA SOUCHE ET PROCÉDÉ DE CULTURE DE PLANTES UTILISANT CETTE COMPOSITION

(30) Priority: 12.05.2022 IT 202200009890
(43) Date of publication of application: 15.11.2023
(73) Proprietor: Associazione Marsicana Produttori Patate - Società Cooperativa Agricola, 67043 Celano (AQ) (IT)
(72) Inventor: Pellegrini, Marika, 03033 ARPINO (FR) (IT); Djebaili, Rihab, 67100 L'AQUILA (AQ) (DZ); Spera, Daniela M., 67100 L'AQUILA (AQ) (IT); Del Gallo, Maddalena, 00061 ANGUILLARA SABAZIA (RM) (IT); Kitouni, Mahmoud, 25000 CONSTANTINE (DZ); Di Giammatteo, Vittorio, 67051 AVEZZANO (AQ) (IT); Nucci, Mario, 67041 AIELLI (AQ) (IT)
(74) Representative: Negrini, Elena

(56) References cited:
- NEWS DESK: "Selenium-enriched potatoes from Peter Keogh - FoodBev Media", 20 February 2009 (2009-02-20), XP093002943, Retrieved from the Internet <URL:https://www.foodbev.com/news/selenium-enriched-potatoes-from-peter-keogh/> [retrieved on 20221129]
- DJEBAILI RIHAB ET AL: "Actinomycete Strains Isolated from Saline Soils: Plant-Growth-Promoting Traits and Inoculation Effects on Solanum lycopersicum", SUSTAINABILITY, vol. 12, no. 11, 5 June 2020 (2020-06-05), pages 4617, XP093002897, DOI: 10.3390/su12114617
- DJEBAILI RIHAB ET AL: "Characterization of Plant Growth-Promoting Traits and Inoculation Effects on Triticum durum of Actinomycetes Isolates under Salt Stress Conditions", SOIL SYSTEMS, vol. 5, no. 2, 10 April 2021 (2021-04-10), pages 26, XP093002879, DOI: 10.3390/soilsystems5020026
- FRANCO-CORREA M ET AL: "Evaluation of actinomycete strains for key traits related with plant growth promotion and mycorrhiza helping activities", APPLIED SOIL ECOLOGY, ELSEVIER, AMSTERDAM, NL, vol. 45, no. 3, 1 July 2010 (2010-07-01), pages 209 - 217, XP027111788, ISSN: 0929-1393, [retrieved on 20100523]

## Description

The present invention relates to the field relating to agriculture and cultivation in general and relates to a strain of Streptomyces, to a composition comprising said strain, to a method of using said composition to produce plant products, and to products obtained using the composition or the implementation of the method.

Various strains of Streptomyces are known for agricultural use and intended, for example, to control fungal, bacterial, or nematode pests or to produce flavorings such as vanillin.

An object of the present invention is to propose a strain of Streptomyces, a composition comprising said strain and a method of using said composition to produce plant products by exploiting the phosphorus locked to the growing medium and not assimilable or not directly available to the development and growth of said plants.

A further purpose is to propose plant products with leaves and/or tubers having the optimal phosphorous concentration, even without additional phosphorous inputs, thanks to said strain, composition, and method.

A further scope is to propose plant products enriched in trace elements such as iodine, selenium, zinc, and others thanks to said strain, composition, and method.

### SUMMARY OF THE INVENTION

The strain of Streptomyces albidoflavus object of the present invention is deposited according to the Budapest Treaty at Leibniz-Institut DSMZ-Deutsche Sammlung von Mikroorganismen und Zellkulturen Gmbh in Braunschweig DE and identified by reference DH12 DSM 34155.

This strain was found in a saline desert environment of a geographical area designated as Djendli sebkha, in Northeast Algeria (and located in the coordinates 35°43'15" N; 06°32'23" E).

The composition object of the present invention comprises Streptomyces albidoflavus strain DH12 DSM 34155 in mycelium and/or, preferably, spore form.

The composition may be liquid or solid in particles, microparticles and/or nanoparticles.

In the liquid composition, said spores are dispersed in an aqueous liquid. Such a liquid composition may optionally comprise one or more of: growth factors, agents intended to facilitate dispersion of the spores in the liquid, agents intended to promote adhesion of the spores to solids, for example seeds, tubers or leaves, even when the liquid fraction has evaporated without causing the seeds to stick, or other products intended to facilitate application of the composition or to improve the growth of the plants or to protect them from pests.

In the solid composition, the spores are mixed with carriers (carriers) and/or inert materials, e.g., comprising at least one of sand, zeolite, perlite, clay, pumice or other igneous materials, fossil sand, limestone, mud, inert or inert plant derivatives such as peat, bark, sapwood, heartwood, straw, cob, wherein such carriers and/or inert materials are in powdered fragments and/or chopped.

In both cases of liquid or solid composition, said composition may optionally comprise at least one of:
- adhesion agents, such as starch for example to facilitate adhesion of spores to seeds or other parts of the plant
- adjuvants, such as Neem oil.
- soil conditioners, such as manure or poultry manure.
- pesticides, such as azoxystrobin.
- dispersants, such as maleic acid, tween 20, tween 80 or surfactants.
- trace elements, such as zinc.
- biostimulants, such as mycorrhizae and/or algae and plant extracts.

Such composition in dry or water-dispersed form has an optimal spore density of 10⁶-10⁸ spores per mL of the composition and, if in concentrated liquid form, is to be diluted for use, preferably 10⁹-10¹⁰.

The method for cultivating a vegetable, which is an object of the present invention, provides for the use of the above-described composition and comprises a step of inoculating the strain of Streptomyces albidoflavus reference DH12 DSM 34155 into the vegetable carried out by placing, prior to sowing or during sowing, respective seeds, tubers, or other propagation means, in contact with the composition. Preferably, this method involves using the composition in liquid form with a concentration of 10⁵-10⁸ spores per mL.

Alternatively, or in addition to the step described above, the method comprises a step of applying the composition to the soil and/or foliar application of said composition after sowing and/or during the vegetative development of the plant.

The method comprises using a composition in liquid form with a concentration of between 10⁵ and 10⁹ spores per mL, preferably of preferably a spore density of between 10⁶ and 10⁸ spores per mL, and sprinkling said liquid composition in an amount of between 200 and 250 (preferably about 220) L/ha for each sprinkling cycle, wherein the total number of cycles may depend on the type of plant and/or soil, environmental and/or meteorological factors or other parameters.

In the case of soil containing at least a percentage of phosphorous that is not directly available for plant development, the method involves using the composition, fertilizers, soil conditioners, and any other crop products that are free or almost free of phosphorous and phosphorous-containing substances.

The plant products (e.g., horticultural products, preferably potatoes), obtained by the above-described method comprise an amount of phosphorus on the dry matter obtained from leaves or tubers of the plant of between about 15 and 35 µg/g or about 15 and 30 µg/g, respectively.

It has been observed that such a plant product, in particular the potato tuber, can also be enriched with an optimal quantity of microelements such as zinc, selenium, iodine, etc.; presumably, this surprising result is also achieved because of a biostimulant action on the root system of said plant product carried out by Streptomyces albidoflavus, strain DH12, DSM 34155 and by the method of the invention.

### DETAILED DESCRIPTION OF THE INVENTION

The present invention relates to an agronomic procedure for obtaining horticultural products with a phosphorus content obtained exclusively by mobilizing soil mineral resources by the application of a Streptomyces albidoflavus (strain DH12, DSM 34155) bacterial strain with phosphate solubilizing properties. The bacterial strain Streptomyces albidoflavus (strain DH12, DSM 34155) also exhibits plant growth-promoting capabilities through direct and indirect mechanisms on the plant that improve its physiological and phytosanitary status.

Phosphorus (P) is a major limiting nutrient that influences plant growth and establishment in terrestrial ecosystems (Ahmad et al., 2018). The redistribution of P varies greatly depending on soil and climate characteristics; it is greater in arid areas than in areas with moderate or high moisture supply, and P redistribution is a major determinant of soil P levels (de-Bashan et al., 2022). In addition, P availability is lower in dry soils, compared to wetter soils, due to alkaline pH values, abundant calcium carbonate content, and the presence of P-binding compounds; while the amount of inorganic P is higher, organic matter, microbial mobilization, and phosphatase activities are lower in dry areas than in wetter areas (de-Bashan et al., 2022). In soil, P is present in a range of inorganic (Pi) and organic (Po) forms, which determine its bioavailability and the potential risk of P loss. Most forms of P in agricultural soils are Pi, which plants cannot absorb or utilize. This situation requires continuous phosphorus fertilization campaigns to ensure optimal agricultural production (Pellegrini et al., 2021). Applying these products represents a massive cost for farmers and contributes to environmental pollution. Phosphate supplementation is only achieved through continuous fertilization campaigns and applied chemical compounds, which lead to a stratification of inaccessible forms of phosphates in soils. Continuous cultivation campaigns lead to an accumulation of Pi, which in turn causes phosphate stratification and contamination of soils and water with consequent environmentally damaging secondary processes such as eutrophication.

To counteract this, microorganism-based agronomic practices (PGPM) have been developed in recent decades to sustainably ensure production quality. The promotion of plant growth and development attributed to PGPMs is also due to their ability to solubilize soil nutrients, such as phosphorous. PGPM with this capacity are known as phosphate-solubilizing microorganisms (PSM) and can convert inorganic and inaccessible forms of phosphate uptake (PO₄³⁻) into available forms (e.g., HPO₄²⁻). This conversion is usually mediated by the organic acids produced by PSMs, which induce the release of phosphates from insoluble complexes using various approaches (i.e., lowering soil pH, chelation, and mineralization). The inoculation of PSM, as an alternative to synthetic chemical fertilizers, is a viable alternative to support plant nutrition by mobilizing locked-up phosphorous, preserving soils from losses of this essential chemical element.

It is, therefore, an object of the present invention to provide an agronomic procedure for obtaining horticultural product seeds inoculated with a bacterial strain having phosphate solubilizing properties and improving the physiological and phytosanitary state of the plant, comprising the following steps:
Dispersion of the spore solution of the strain Streptomyces albidoflavus (strain DH12, DSM 34155) at a density of 10⁶-10⁸ spores per milliliter on the seeds or seed tubers prior to or during tuber sowing.

Cultivation of the plants following sowing as per point 1 in the complete absence of phosphate fertilization.

Harvesting of the product from the plants following cultivation as per point 2.

According to the present invention, it is also possible to add to the spore solution a spore activator product or other bacterial or fungal strains with recognized plant growth promotion or plant pathogen biocontrol properties. Thus, the further object of the present invention is the combination of biostimulant and/or biofertiliser products.

The present invention also relates to an agronomic procedure for obtaining horticultural product seeds inoculated with a bacterial strain having phosphate solubilizing properties and improving the physiological and phytosanitary state of the plant. The bacterial strain used is available from the database Deutsche Sammlung von Mikroorganismen und Zellkulturen (DSMZ, Braunschweig, Germany) - Streptomyces albidoflavus (strain DH12, DSM 34155) - and the invention can be introduced into the standard horticultural production process with open field cultivation. The process steps can be schematized as follows:
Bacterial strain cultivation: preparation of the bacterial inoculum, the Streptomyces albidoflavus strain (strain DH12, DSM 34155) can be cultivated in generic culture media widely described in the literature and under culture conditions of continuous agitation and constant temperature of 25-30°C for 5-7 days depending on the chosen culture medium.

Dilution of the formulation: the formulation, generally supplied as a concentrated solution, 10⁹-10¹⁰ spores per mL, can be diluted with irrigation water or mixed with solid inert compounds.

Inoculation: inoculation takes place before or during sowing. It involves contact of the solution or inert solid product containing spores of the Streptomyces albidoflavus (strain DH12, DSM 34155) with a density of 10⁶-10⁷ spores per mL of solution with the seeds or seed tubers.

Horticultural cultivation: it is conducted following current agronomic practice but without phosphate fertilization. The strain, as described, can solubilize Pi, normally present in excess in soils; the strain, therefore, supplies the amount of phosphorous required by the plant, providing assimilable forms to the plant's root system.

### EXAMPLE

The present example demonstrates the ability to treat seed tubers of Solanum tuberosum with the Streptomyces albidoflavus strain (strain DH12, DSM 34155) with a density of 10⁶ spores per mL to mobilize phosphorus in the soil and make it available for plant uptake. The experimental trials were conducted in 2021 at three farms in the Fucino area (Abruzzo, Italy). The farms, located in the following localities: (A); (B); (C), have an extension of 1 hectare, and the experimental trials were conducted following a split-plot experimental design, contrasting the experimental control condition (untreated seed tubers) with the treated experimental condition (seed tubers treated by sprinkling with a liquid solution of Streptomyces albidoflavus (strain DH12, DSM 34155) with a density of 10⁶ spores per mL obtained by dilution with irrigation water before sowing). At farm C, another experimental condition was also introduced, which involved treating the seed tubers during the sowing procedures by loading the liquid solution of Streptomyces albidoflavus (strain DH12, DSM 34155) with a density of 10⁶ spores per mL into the seed drill and sprinkling it through the nozzles of the coulter bar over the tuber once it had been placed in the seed bed. All experimental fields were managed in the absence of phosphate fertilization. When the tubers were ripe, the plant and potato samples collected from the different experimental blocks were labelled and transferred to the Agro-Environmental Microbiology laboratory for evaluation of the following parameters:
Dry matter, the percentage dry weight of the tubers was obtained by obtaining 2 cm × 2 cm cubes from the samples, transferring the obtained matrix by drying in an oven at 80°C until a stationary weight was reached (approximately 76 hours) and calculated using the following formula: "dry matter %=" "Net weight in grams dried matrix" / "Net weight in grams fresh matrix" " × 100"

Phosphorus content, the tubers, and leaves of the control and treated plants, previously dried and homogenized using a mortar to obtain a fine powder (Gerdel, 1928), were digested using a special solution. The extracts obtained were subjected to spectrophotometric quantification of phosphorus as follows:
Sample digestion, the fine powder of each sample was treated with 3 mL of HNO₃ (CAS 7697-37-2) 65%: HCl (CAS 7647-01-0) 37% (1:3, v/v) solution. The mixtures were digested in a thermostatic ultrasonic bath at 95 °C for 4-5 h until the complete dissolution of each sample (Uddin et al., 2016). During digestion, the inner walls of the beakers were washed with 2 mL of deionized water to prevent sample loss, and, in the last part of the digestion process, the samples were filtered with Whatman 42 filter paper (particle retention 2.5 µm). Finally, deionized water was added to obtain a final volume of 17 mL.

Determination of phosphorus content, measured according to the method of Olsen and Sommers (Olsen & Sommers, 1982) as follows: To a 50 mL beaker containing 1 mL of digested extract obtained as described above, 10 mL of ammonium molybdate (CAS 12027-67-7) 12 mM and 1 mL of stannous chloride (CAS 7772-99-8) 5 mM were added. The mixtures were made up to 50 mL volume with distilled water, and the blue color intensity was measured spectrophotometrically at a wavelength of 620 nm (Thermo Scientific^{™} Multiskan^{™} GO Microplate Spectrophotometer). The standard calibration curve was performed with a solution of KH₂PO₄ (CAS 7778-77-0) (y = 0.2555x + 0.021).

The tests were conducted in triplicate, taking 3 biological replicates from the overall potato and leaf samples sampled in the field and proceeding with processing and quantification independently.

The results obtained were analyzed for normality; from Table I, given at the end of the description, the results for dry matter show a non-normal distribution; therefore, they were analyzed using non-parametric tests (multiple pairwise comparisons using the Dunn's procedure / Two-sided test:). On the other hand, the results for phosphorus content (both plant and tuber) are normal, and the data were analyzed using parametric tests (ANOVA followed by Fisher's LSD post-hoc test).

Table II, at the end of the description, shows the dry matter results recorded for the control and treated experimental conditions (pre-sowing and at sowing for farm C).

Generally, results showed that the treated experimental conditions recorded a higher dry matter value. However, according to Dunn's test, only the difference between control and treated on farm A was statistically significant (p < 0.05). The agronomic treatment would therefore appear to have a non-significant influence on the amount of dry matter in the tuber and a variable influence depending on the production site. The multiple comparisons also showed that the differences between the groups were not very marked, except for the contrast between the control of farm B, with the lowest dry matter value, and the treatment of farm A, with the highest dry matter value.

The leaf and tuber samples were analyzed for phosphorus content to be able to define the differences between the amount of phosphorus assimilated and translocated compared to the treated. Table III at the end of the description shows the results obtained. From the results and statistical comparison, the leaves and tubers of the experimental conditions have a lower phosphorus content than those of the control conditions. This statistically significant difference (p < 0.05) indicates the effective ability of the treatment to improve the mobilization capacity of assimilable phosphorus and make it available for plant uptake. Having been enriched in the absence of phosphate-based chemical fertilization the tuber was enriched with phosphorus already present in the soils and mobilized by the activity of the bacterial strain used with Pi-solubilizing properties. Laboratory tests carried out at the Agro-Environmental Microbiology Laboratory of the University of L'Aquila also revealed interesting characteristics of the strain used, such as: indolacetic acid production capacity, ACC deaminase, ammonia production, hydrocyanic acid production, enzyme production, biocontrol of phytopathogenic fungal and bacterial strains. Therefore, based on what has been achieved and the results shown in scientific publications in international scientific journals, the treatment is also considered useful for cultivating other horticultural plant species.

**Table I - Shapiro-Wilk normality test result with 5% significance level (α 0.05).**

| | Dry weight | P leaves | P tubers |
|---|---|---|---|
| W | 0.973 | 0.830 | 0.888 |
| p-value (bilateral) | 0.796 | 0.002 | 0.020 |

**Table II - Results of dry matter content of Solanum tuberosum tubers.**

| | Dry weight (%) | p-value comparison control vs treated | Statistical groups |
|---|---|---|---|
| A control | 16.07 ± 1.59 | 0.048 | a, b |
| A treated | 18.55 ± 0.47 | | c |
| B control | 14.09 ± 0.80 | 0.065 | a |
| B treated | 16.38 ± 0.23 | | a, b, c |
| C control | 14.48 ± 0.92 | | a, b |
| C treated pre-sowing | 16.38 ± 1.10 | 0.130 | a, b, c |
| C treated during sowing | 16.90 ± 0.94 | 0.076 | b, c |

**Table III - Results of phosphorus content (µg / g dry matter) obtained for leaves and tubers of Solanum tuberosum.**

| | P leaves (µg / g) | P tubers (µg / g) |
|---|---|---|
| A control | 11.10 ± 1.66 d | 11.70 ± 0.69 d |
| A treated | 17.24 ± 1.36 b | 20.62 ± 0.23 b |
| B control | 10.96 ± 0.55 d | 11.56 ± 0.50 d |
| B treated | 17.60 ± 0.47 b | 16.64 ± 1.27 c |
| C control | 13.24 ± 0.70 c | 11.39 ± 0.88 d |
| C treated pre-sowing | 29.35 ± 0.60 a | 25.76 ± 1.19 a |
| C treated during sowing | 30.96 ± 0.23 a | 17.18 ± 4.50 c |
| Least significant difference | 1.63 | 3.30 |

## Claims

1. Streptomyces albidoflavus strain deposited under the Budapest Treaty at Leibniz Institut DSMZ-Deutsche Sammlung von Mikroorganismen und Zellkulturen Gmbh in Braunschweig DE and identified by the reference DH12 DSM 34155.

2. Composition comprising the Streptomyces albidoflavus strain DH12 DSM 34155 of claim 1 **characterized in that** such strain is in the form of spores and/or mycelium dispersed in an aqueous liquid and/or dry mixed with carriers and/or inert, including at least one among sand, zeolite, perlite, clay, pumice or other igneous materials, diatomaceous earth, limestone, mud, inert or inert plant derivatives such as peat, bark, sapwood, heartwood, straw, corncobs, where such carrier and/or inert are powdered, in fragments and/or ground.

3. Composition according to claim 2 **characterized in that** it comprises at least one among adhesion agents, such as starch for example to facilitate the adhesion of the spores to seeds or other parts of a plant; adjuvants, such as Neem oil; soil conditioners, such as manure or chicken manure; pesticides, such as azoxystrobin; dispersants, such as maleic acid, tween 20, tween 80 or surfactants; micronutrients, such as zinc; biostimulants, such as mycorrhizae and/or algae and plant extracts.

4. Composition according to claim 2 or 3 **characterized in that** it is in dry form or dispersed in water with a spore density ranging from 10⁶ to 10¹⁰ spores per mL of composition.

5. Method of cultivation of a plant using the composition of any one of claims 2 to 4 comprising the step of putting, before sowing or during sowing, a plant or its seeds, tubers, or other means of propagation, in contact with the composition or inoculating said plant or seeds, tubers or other propagation means with said strain of the composition.

6. Method according to claim 5 **characterized in** using a composition in liquid form with a concentration ranging from 10⁵ to 10⁸ spores per mL.

7. Method according to claim 5 **characterized in that** it comprises the phase of application on the ground and/or of foliar application of the composition after sowing and/or during the vegetative development of the plant.

8. Method according to claim 7 **characterized in** using a composition in liquid form with a concentration ranging from 10⁵ to 10⁹ spores per mL and sprinkling said liquid composition in an amount comprised between 200 and 250 L/ha for each sprinkling cycle.

9. Method according to any one of claims 5 to 8 **characterized in** providing, in the case of soil comprising at least a percentage of phosphorus not directly available for the development of the plant, to use the composition, fertilizers or soil conditioners and the like free or nearly free of phosphorus.

## Patentansprüche

1. Streptomyces albidoflavus Stamm, hinterlegt im Budapest Vertrag am Leibniz Institut DSMZ-Deutsche Sammlung von Mikroorganismen und Zellkulturen GmbH in Braunschweig, DE und identifiziert über die Referenz DH12 DSM 34155.

2. Zusammensetzung, die den Streptomyces albidoflavus Stamm DH12 DSM 34155 des Anspruchs 1 enthält, **dadurch gekennzeichnet, dass** solch ein Stamm die Form von Sporen und/oder Myzel aufweist, aufgelöst in einer wässrigen Flüssigkeit und/oder trocken gemischt mit Trägern und/oder inerten Materialien, enthaltend mindestens eines der Folgenden: Sand, Zeolith, Perlit, Ton, Bimsstein oder andere magmatische Materialien, Kieselgur, Kalkstein, Lehm, inerte Materialien oder inerte Pflanzenderivate wie Torf, Rinde, Splintholz, Kernholz, Stroh, Maiskolben, wobei derartige Träger und/oder inerte Materialien pulvrig, bruchstückig und/oder gemahlen sind.

3. Zusammensetzung gemäß Anspruch 2, **dadurch gekennzeichnet, dass** sie mindestens ein Haftmittel umfasst, wie Stärke, um beispielsweise die Haftung der Sporen an Samen oder anderen Teilen einer Pflanze zu erleichtern; Hilfsstoffe, wie Neemöl; Bodenverbesserungsmittel, wie Dung oder Hühnerdung; Pestiziden, wie Azoxystrobin; Dispergiermittel wie Maleinsäure, Tween 20, Tween 80 oder Tenside; Mikronährstoffe, wie Zink; Biostimulatoren wie Mycorrhizae und/oder Algen und Pflanzenextrakte.

4. Zusammensetzung gemäß Anspruch 2 oder 3, **dadurch gekennzeichnet, dass** sie in trockener Form oder in Wasser aufgelöst vorliegt, mit einer Sporendichte im Bereich von 10⁶ bis 10¹⁰ Sporen pro mL der Zusammensetzung.

5. Verfahren zur Züchtung einer Pflanze unter Verwendung der Zusammensetzung gemäß einem der Ansprüche 2 bis 4, umfassend den Schritt des in Kontaktbringens, vor der Aussaat oder während der Aussaat, einer Pflanze oder ihrer Samen, Knollen oder anderer Vermehrungsmittel, mit der Zusammensetzung oder Impfen der Pflanze oder Samen, Knollen oder anderer Vermehrungsmittel mit dem Stamm der Zusammensetzung.

6. Verfahren gemäß Anspruch 5, **dadurch gekennzeichnet, dass** eine Zusammensetzung in flüssiger Form verwendet wird mit einer Konzentration im Bereich von 10⁵ bis 10⁸ Sporen pro mL.

7. Verfahren gemäß Anspruch 5, **dadurch gekennzeichnet, dass** es eine Phase der Anwendung der Zusammensetzung auf dem Boden und/oder Anwendung auf dem Blatt nach der Aussaat und/oder während der vegetativen Entwicklung der Pflanze umfasst.

8. Verfahren gemäß Anspruch 7, **dadurch gekennzeichnet, dass** eine Zusammensetzung in flüssiger Form mit einer Konzentration im Bereich von 10⁵ bis 10⁹ Sporen pro mL verwendet wird und Sprenkeln der flüssigen Zusammensetzung in einer Menge zwischen 200 und 250 L/ha für jeden Besprenkelungszyklus.

9. Verfahren gemäß einem der Ansprüche 5 bis 8, **dadurch gekennzeichnet, dass** im Falle von Erde, die mindestens einen Prozentsatz von Phosphor enthält, der nicht direkt für die Entwicklung der Pflanze zugänglich ist, die Verwendung der Zusammensetzung, Düngemittel oder Bodenverbesserungsmittel und dergleichen frei von oder nahezu frei von Phosphor vorgesehen ist.

## Revendications

1. - Souche de Streptomyces albidoflavus déposée au titre du Traité de Budapest auprès de l'Institut Leibniz DSMZ-Deutsche Sammlung von Mikroorganismen und Zellkulturen Gmbh (Collection allemande de micro-organismes et de cultures cellulaires Gmbh) à Braunschweig DE et identifiée par la référence DH12 DSM 34155.

2. - Composition comprenant la souche de Streptomyces albidoflavus DH12 DSM 34155 selon la revendication 1, **caractérisée par le fait qu'**une telle souche est sous forme de spores et/ou de mycélium dispersés dans un liquide aqueux et/ou mélangés à sec avec des supports et/ou une matière inerte, comprenant au moins l'un parmi le sable, la zéolite, la perlite, l'argile, la pierre ponce ou d'autres matières ignées, la terre de diatomées, le calcaire, la boue, une matière inerte ou des dérivés végétaux inertes tels que la tourbe, l'écorce, l'aubier, le bois de cœur, la paille, les épis de maïs, où de tels support et/ou matière inerte sont en poudre, en fragments et/ou broyés.

3. - Composition selon la revendication 2 **caractérisée par le fait qu'**elle comprend au moins l'un parmi des agents d'adhésion, tels que l'amidon par exemple pour faciliter l'adhésion des spores aux graines ou à d'autres parties d'une plante ; des adjuvants, tels que l'huile de Neem ; des amendements de sol, tels que du fumier ou du fumier de poulet ; des pesticides, tels que l'azoxystrobine ; des dispersants, tels que l'acide maléique, le tween 20, le tween 80 ou des tensio-actifs ; des micronutriments, tels que le zinc ; des biostimulants, tels que des mycorhizes et/ou des extraits d'algues et de plantes.

4. - Composition selon l'une des revendications 2 ou 3 **caractérisée par le fait qu'**elle est sous forme sèche ou dispersée dans l'eau avec une densité de spores allant de 10⁶ à 10¹⁰ spores par mL de composition.

5. - Procédé de culture d'une plante à l'aide de la composition selon l'une quelconque des revendications 2 à 4 comprenant l'étape de mettre, avant le semis ou pendant le semis, une plante ou ses graines, tubercules ou autres moyens de propagation, en contact avec la composition ou d'inoculer ladite plante ou graines, tubercules ou autres moyens de propagation avec ladite souche de la composition.

6. - Procédé selon la revendication 5 **caractérisé par** l'utilisation d'une composition sous forme liquide avec une concentration allant de 10⁵ à 10⁸ spores par mL.

7. - Procédé selon la revendication 5 **caractérisé par le fait qu'**il comprend la phase d'application au sol et/ou d'application foliaire de la composition après le semis et/ou pendant le développement végétatif de la plante.

8. - Procédé selon la revendication 7 **caractérisé par** l'utilisation d'une composition sous forme liquide avec une concentration allant de 10⁵ à 10⁹ spores par mL et l'arrosage de ladite composition liquide dans une quantité comprise entre 200 et 250 L/ha pour chaque cycle d'arrosage.

9. - Procédé selon l'une quelconque des revendications 5 à 8 **caractérisé par le fait qu'**il prévoit, dans le cas de sol comprenant au moins un pourcentage de phosphore non directement disponible pour le développement de la plante, d'utiliser la composition, les engrais ou amendements de sol et similaires exempts ou quasiment exempts de phosphore.
